# EUROPEAN PATENT SPECIFICATION

(11) **EP 1 326 599 B1**
(45) Date of publication and mention of the grant of the patent: **13.12.2006**
(21) Application number: 01976452.1
(22) Date of filing: 15.10.2001
(51) Int. Cl.: A61K 31/351, A61P 31/04

(54) **Use of aivlosin for treating or preventing Lawsonia infections in pigs.**
Verwendung von Aivlosin zur Behandlung und Prevention der Lawsonia-Infektionen in Schweinen.
Utilisation d'aivlosin pour traiter et prévenir des infections de Lawsonia chez les cochons.

(30) Priority: 18.10.2000 GB 0025556
(43) Date of publication of application: 16.07.2003
(73) Proprietor: Eco Animal Health Ltd., Southgate, London N14 6HF (GB)
(72) Inventor: SANDERS, Michael, John, Southgate, London N14 6HF (GB)
(74) Representative: Manaton, Ross Timothy
(86) International application number: PCT/GB2001/004575
(87) International publication number: WO 2002/032233

(56) References cited:
- EP-A- 0 121 328
- JACKS, T.M., ET AL: "3-Acetyl-4''-isovalereyl tylosin for prevention of swine dysentry" AMERICAN JOURNAL OF VETERINARY RESEARCH, vol. 47, no. 11, 1986, pages 2325-2328, XP001053272
- SKELLY, B.J., ET AL: "Prophylactic efficacy of 3-acetyl-4''-isovaleryl tylosin in a mycoplasma gallisepticum-induced airsacculitis infection" AVIAN DISEASES , vol. 30, no. 3, 1986, pages 505-509, XP001053258
- DATABASE CA [Online] CHEMICAL ABSTRACTS SERVICE, COLUMBUS, OHIO, US; OKUYAMA, DAISAKU: "3-Acetyl-4''-isovaleryltylosin, a new animal drug" retrieved from STN Database accession no. 119:194935 HCA XP002190573 & KACHIKU KOKINZAI KENKYUKAIHO (1991), 12, 44-57 ,
- DATABASE CA [Online] CHEMICAL ABSTRACTS SERVICE, COLUMBUS, OHIO, US; LEE, MUN-HAN ET AL: "Studies on efficacy and establishment of withdrawal time of acetyl isovaleryl tylosin tartrate and chlortetracycline combination for bacterial pneumonia therapy in swine: I. Antimicrobial activity of acetyl isovaleryl tylosin tartrate and chlortetracycline combination in vitro" retrieved from STN Database accession no. 130:217665 HCA XP002189926 & SOUL TAEHAKKYO SUUIDAE NONMUNJIP (1997), 22(1), 57-62 ,
- DATABASE CA [Online] CHEMICAL ABSTRACTS SERVICE, COLUMBUS, OHIO, US; LEE, MUN-HAN ET AL: "Studies on efficacy and establishment of withdrawal time of acetyl isovaleryl tylosin tartrate and chlortetracycline combination therapy in swine. III. Establishment of withdrawal time of acetyl isovaleryl tylosin tartrate and chlortetracycline combination" retrieved from STN Database accession no. 130:191458 HCA XP002189927 & SOUL TAEHAKKYO SUUIDAE NONMUNJIP (1998), 23(1), 25-34 ,
- DATABASE CA [Online] CHEMICAL ABSTRACTS SERVICE, COLUMBUS, OHIO, US; CHO, SEUNG-KUN ET AL: "Studies on efficacy and establishment of withdrawal time of acetyl isovaleryl tylosin tartrate and chlortetracycline combination therapy in swine, II. Field efficacy trials of acetyl isovaleryl tylosin tartrate an chlortetracycline combination" retrieved from STN Database accession no. 130:177172 HCA XP002189928 & SOUL TAEHAKKYO SUUIDAE NONMUNJIP (1998), 23(1), 19-24 ,
- S. MCORIST, ET. AL.: "Monitored control programme for proliferative enteropathy on British pig farms" VETERINARY RECORD, vol. 144, 1999, pages 202-204, XP008043238
- S. MCORIST: "Oral administration of tylosin phosphate for treatment and prevention of proliferative enteropathy in pigs" AM. J. VET. RES., vol. 58, no. 2, 1997, pages 136-139,

## Description

The present invention relates to the use of antibiotics as veterinary medicaments for the treatment or prophylaxis of diseases and infections of animals, specifically pigs.

Pigs, especially those which are intensively reared or reared in large-scale operations, have a tendency to suffer from or risk catching a variety of diseases and infections, for example Mycoplasma diseases, Lawsonia infections and swine dysentery. Medicaments have been proposed or used for the treatment of individual diseases or infections of these types. Such medicaments are either, not in general thought to be highly effective in a wide range of diseases or infections or not thought to be effective at low dosage levels. Thus, for example, erythromycin, which is used against Mycoplasma, has no reported effect against swine dysentery or Lawsonia.

Surprisingly, we have now found that the known antibiotic aivlosin (otherwise known as 3-0-acetyl-4"-0 isovaleryl-tylosin), which has previously been used in high doses for the treatment and control of Mycoplasma diseases in poultry, is also effective in the prevention and treatment of Lawsonia infections (ileitis) in pigs.

The present invention therefore provides for the use of aivlosin, as such or as a pharmacologically acceptable acid addition salt, in the preparation of a veterinary medicament for the treatment or prophylaxis of Lawsonia infections in pigs.

It also includes a stable coated composition for addition to animal feed comprising aivlosin in particulate form coated with polyvinyl pyrrolidone.

In British Patent Specification No. 1,539,907 there are disclosed tylosin derivatives having acyl groups in the 3 and 4" positions and acid addition salts thereof, specifically the tartaric, acetic, propionic, citric, succinic, hydrochloric, sulphuric and phosphoric acid addition salts. Amongst the tylosin derivatives specifically disclosed there is 3-0-acetyl-4"-0-isovaleryl-tylosin, which is now commonly known as aivlosin. This compound has the formula 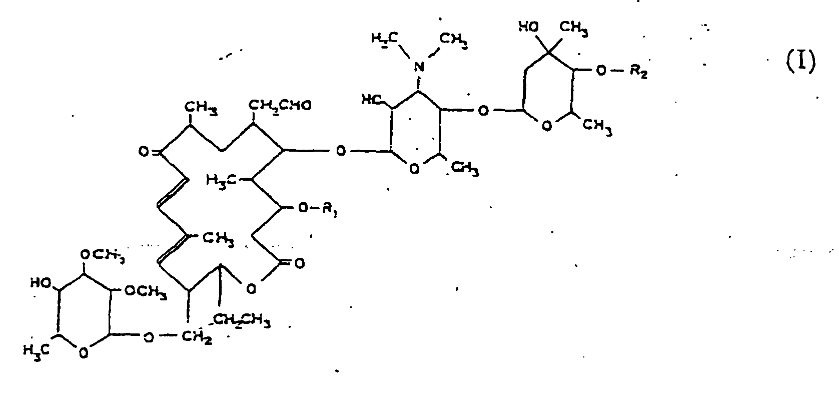 where R1 is acetyl and R2, is isovaleryl. There is also disclosed a process for the production of aivlosin by the biochemical acylation of tylosin or an appropriately partially acylated tylosin by means of an appropriate acylating microorganism of the genus Streptomyces, especially one selected from Streptomyces thermotolerans (ATCC11416), Streptomyces fungicidus subsp. espinomyceticus (ATCC 21574), Streptomyces mycarofaciens (ATCC 21454) and Streptomyces hygroscopicus (ATCC 21582), in the presence of the appropriate acyl donor, especially acetyl CoA, isovaleryl CoA, acetic acid, isovaleric acid, potassium, sodium or ammonium salts of those acids, methanol and ethanol esters of these acids, amides of these acids and α-oxovaleric acid.

The said Specification mentions that the tylosin derivatives can be administered to humans or animals and refers to their activity against a number of gram-positive bacteria, including some drug-resistant bacteria, but it does not specifically refer to the use of the derivatives in the treatment or control of specific diseases or infections of animals, although it does say that they can be employed on humans, livestock, household pets, laboratory animals and poultry and in the enteral, parenteral or topical control of infectious diseases in a similar manner as for known macrolide antibiotic drugs.

In fact, aivlosin on the basis of its initial Japanese marketing registration (No 4 chika AC1771) has to date been marketed and approved for marketing only for the treatment and control of Mycoplasma diseases in pigs and poultry at high doses of 200 to 500 ppm in feed. There should be no reason to suppose that it would be suitable for the treatment and prophylaxis of other infections and diseases of pigs, and in particular other macrolide antibiotics having an effectiveness against Mycoplasma diseases such as erythromycin do not have any effect or any significant effect against other infections of pigs such as those mentioned above. It is, of course, a feature of the approvals schemes which apply in all major countries that a veterinary medicament which is approved for marketing for one specific purpose cannot be marketed or recommended for use for any other specific purpose without a separate authorisation or approval from the relevant Authority. There is thus a strong counter-incentive to the use of even known antibiotics for new veterinary uses.

However, we have now found and confirmed from extensive in vitro and in vivo (animal) trial work that aivlosin and pharmaceutically acceptable acid addition salts thereof are effective in the prevention and treatment of Lawsonia infections in pigs at reasonable dose rates.

Aivlosin is available in free form as a white crystalline powder having a melting point of 180°-184°C, soluble in lower alcohols such as ethanol, ketones such as acetone, ethers such as diethyl ether, esters such as ethyl acetate and aromatic hydrocarbons such as toluene, although it is barely soluble in n-hexane and petroleum ether. It is very soluble in aqueous solutions of pH around and below 7 but less soluble in aqueous solutions of higher pH. Because it is a basic compound it forms acid addition salts, and the use of such salts which are pharmacologically acceptable is also included within the present invention. Acids to form acceptable acid addition salts include inorganic acids such as hydrochloric, sulphuric or phosphoric acid and organic acids such as tartaric, acetic, propionic, citric and succinic acids. Specific examples of acceptable derivatives are aivlosin hydrochloride (melting point 129-133°C) and aivlosin tartrate (melting point 119-122°C). Such derivatives are frequently more watersoluble than aivlosin itself and their use may therefore have formulation advantages.

Aivlosin and appropriate pharmaceutically acceptable acid addition salts thereof can be formulated according to the present invention into veterinary medicaments in known ways, for example to provide compositions for oral, enteral or parenteral administration, by admixing with appropriate solid or liquid carriers and excipients for the adminstration route desired. Conventional ingredients can be used as carriers and excipients, for example water and salt solutions for liquid formulations and silicaceous materials-silica and silicates (such as hydrated magnesium silicate)-, cereal products (such as soybean meal and wheat flour) and other pharmacologically acceptable solids for solid formulations for oral administration. The formulations can also contain further auxiliaries and additives such as minerals, lubricants, preservatives, stabilisers, wetting agents, emulsifiers, buffers and colouring or flavouring materials in a conventional manner. In the prophylaxis or control of Lawsonia infections it is particularly convenient to include the aivlosin or pharmaceutically acceptable acid addition salts thereof as an additive to animal feed or drinking water, but it can be included in an injectable solution, or a tablet, capsule or syrup, if desired.

Aivlosin (as such or in the form of an appropriate acid addition salt such as the tartrate) may be formulated into premixes in various potencies from 1 to 10% by weight. A particularly suitable composition for producing such premixes comprises aivlosin salt, filler such as soybean powder and additives such as hydroxypropyl cellulose and has a potency of 180 to 220 mg/ g.

In order to ensure stability of aivlosin in animal feed which may have been subjected to high-temperature processing for pelleted or extruded feed it is desirable to provide a coated aivlosin (as such or in the form of an appropriate acid addition salt such as the tartrate) in particulate form coated with polyvinylpyrrolidone. Suitable proportions by weight are in the range active ingredient: polyvinyl pyrrolidone 50:1 to 1:1. Inert fillers and other ingredients may be present in such compositions, the overall polyvinylpyrrolidone concentration being preferably 0.1 to 10% by weight.

The veterinary medicament formulations can also contain further active ingredients useful in the treatment of Lawsonia infections in pigs, such as further antibiotics, in particular tetracycline antibiotics, for that purpose.

The veterinary medicament formulations for use either as feed additives or as directly administered preparations may contain any convenient proportion of aivlosin for example from 1% or less to 90% or more, by weight. Liquid formulations typically contain 50 to 90% by weight, whereas solid formulations typically contain 1 to 25% by weight.

Such formulations may for example be administered in feed at a rate of 40 to 120 ppm by weight (40-120 g per 1,000 kg of feed) for a period of time long enough to control or treat the disease successfully, for example 7 to 14 days. For example, figures of 40 to 100 or 50 to 80 ppm may be used. A rate of 50 ppm for 10 days is usually effective in controlling the disease and a rate of 100 ppm for 10 days, is usually very successful in treating it.

When aivlosin or a derivative is directly administered for treatment or control of Lawsonia infections, the adminstration levels based on body weight may be in the range 1 to 8, preferably 1 to 5mg/kg body weight/day.

The following Examples, in which parts are by weight, illustrate the use of aivlosin in the manufacture of veterinary medicaments or preparations according to the invention.

### Example 1

20 parts of aivlosin API (active pharmaceutical ingredient) made into a solution in water is mixed with 80 parts of soybean meal, and the mixture is spray dried to give a solid additive for feedstuff containing 200 kg aivlosin activity per 1000 kg. This formulation can be added to pig feed to provide an infeed concentration of aivlosin of 25 to 200 g aivlosin per 1000 kg final feed.

### Example 2

25 parts of aivlosin 20% is mixed with 50 parts of hydrated magnesium silicate (an inert silica), 24 parts of wheat feed flour and 1 part of liquid paraffin EP as a powder blend to give a solid additive for feedstuff containing 50 kg aivlosin activity per 1000 kg. This formulation can be used in pig feed as in Example 1.

### Example 3

5 parts of aivlosin 20% as used in Example 2 is mixed with 40 parts of hydrated magnesium silicate, 54 parts of wheat feed flour and 1 part of liquid paraffin EP as a powder blend to give a solid additive for feedstuff containing 10 kg aivlosin activity per 1000 kg. This formulation can be used in pig feed as in Example 1.

### Example 4

Aivlosin is dissolved in water to provide an aqueous solution containing 80-90% aivlosin activity for use in drinking water for pigs. This formulation can be added to drinking water to provide aivlosin concentrations in drinking water in the range 25 to 100 g per 200 litres of drinking water.

### Example 5

Aivlosin API containing more than 80% w/w aivlosin tartrate was mixed into an 850 kg batch comprising:

| | |
|---|---|
| Aivlosin API | 163-169 kg |
| Hydroxypropyl cellulose. Ph. Eur. | 8.2-8.5 kg |
| Water, Ph. Eur. | 800-1200 litres |
| Non-fat soybean powder | 720 kg |

The batch was processed and the water was removed during processing. The input of aivlosin API was adjusted for content value of free base, determined by HPLC, of the raw material to achieve a final product bioassay potency of 180-220 mg/g. The product (AIVLOSIN FG 200), which could also be produced in other batch sizes, was suitable for manufacturing aivlosin premixes in various potencies from 1 % to 10%.

### Example 6

Coated aivlosin formulations possessing stability in animal feed after high-temperature processing for pelleted or extruded feed were produced in batches of 1000 kg (although other batch sizes could be used) from the following ingredients:

| | |
|---|---|
| AIVLOSIN FG 200 (see Example 5) | 250.0 kg |
| Paraffin, Light Liquid, Ph. Eur. | 10.0 kg |
| Wheat feed flour | 240.0 kg |
| Polyvinylpyrrolidone | 10.0kg-100. 0 kg |
| Sepiolite | to 1000.0 kg |

### Trial Results

Lawsonia infections (ileitis or proliferative porcine enteropathy) in pigs are caused by the pathogen Lawsonia intracellularis, which was isolated only some six years ago and is a bacteria residing in the cells of the intestinal wall of the lower small intestine of pigs. To date, few antimicrobials have been recognised as effective in preventing and treating the disease, which is widespread throughout the world in its incidence and is of considerable economic importance in pig rearing and breeding. Extensive trial work by us both in vitro and in vivo on pigs have shown that aivlosin is very effective in treating the disease and preventing it from spreading further in an infected environment. The following Table 1 shows clinical results of an aivlosin porcine proliferative enteropathy efficacy study carried out by us.

**Table 1**

| | **Mortality** | **Lesion** | **Lesion** |
|---|---|---|---|
| **Group** | **Rate** | **Incidence** | **Severity** |
| | **(%)& [n]** | **(%)** | **(ins.)** |
| Control | 15.1 [3] | 80.0 | 43.1 |
| Aivlosin 50ppm | 13.3 [4] | 73.3 | 36.2 |
| Aivlosin 100ppm | Nil | 33.3* | 3.15* |

| | | | |
|---|---|---|---|
| *Statistically significant [P < 0. 001] from other groups. | | | |

There was also considerable improvement in feed intake, weight gain and feed efficiency in treatment groups with aivlosin. For all these production parameters aivlosin performed well.

Aivlosin at an inclusion rate of 50 grams per tonne (1000kg) of feed (50 ppm), provided for 10 days was effective in controlling the disease, while at 100 ppm for 10 days the outbreak was very successfully treated.

## Claims

1. The use of 3-O-acetyl-4"-0-isovaleryl-tylosin or a pharmacologically acceptable acid addition salt thereof for the preparation of a veterinary medicament for the treatment or prophylaxis of *Lawsonia* infections in pigs.

2. The use as claimed in claim 1, wherein the veterinary medicament is an additive to feed or drinking water.

3. The use as claimed in claim 1 or 2, wherein the medicament is formulated as a feed for pigs, containing 25 to 200 ppm of 3-O-acetyl-4"-O-isovaleryl-tylosin or a pharmacologically acceptable acid addition salt thereof.

4. The use as claimed in claim 3 wherein the concentration of 3-O-acetyl-4"-O-isovaleryl-tylosin in the feed is 40 to 120 ppm.

5. The use according to any of the claims 2 to 4, wherein the feed additive is formulated as a stable coated composition in particulate form coated with polyvinylpyrrolidone.

6. The use according to claim 1 or claim 2, wherein the medicament is formulated to be administered to pigs in their drinking water, at a concentration in the range 25 to 100g per 200 litres.

7. The use according to claim 1, wherein the medicament is adapted to be administered to pigs at a dosage in the range 1 to 8 mg/kg body weight/day.

## Patentansprüche

1. Verwendung von 3-O-Acetyl-4"-O-isovaleryltylosin oder einem pharmazeutisch unbedenklichen Säureadditionssalz davon zur Herstellung eines Tierarzneimittels zur Behandlung oder Prophylaxe von *Lawsonia*-Infekionen bei Schweinen.

2. Verwendung nach Anspruch 1, bei der das Tierarzneimittel ein Futter- oder Trinkwasserzusatz ist.

3. Verwendung nach Anspruch 1 oder 2, bei der das Arzneimittel als Schweinefutter mit 25 bis 200 ppm 3-O-Acetyl-4"-O-isovaleryltylosin oder einem pharmazeutisch unbedenklichen Säureadditionssalz davon formuliert ist.

4. Verwendung nach Anspruch 3, bei der die Konzentration von 3-O-Acetyl-4"-O-isovaleryltylosin im Futter 40 bis 120 ppm beträgt.

5. Verwendung nach einem der Ansprüche 2 bis 4, bei der der Futterzusatz als stabile beschichtete Zusammensetzung in Teilchenform mit Polyvinylpyrrolidon-Überzug formuliert ist.

6. Verwendung nach Anspruch 1 oder 2, bei der das Arzneimittel zur Verabreichung an Schweine in deren Trinkwasser in einer Konzentration von 25 bis 100 g pro 200 Liter formuliert ist.

7. Verwendung nach Anspruch 1, bei der das Arzneimittel auf die Verabreichung an Schweine in einer Dosierung im Bereich von 1 bis 8 mg/kg Körpergewicht/Tag abgestellt ist.

## Revendications

1. Utilisation de la 3-O-acétyl-4"-O-isovaléryl-tylosine ou d'un sel d'addition à un acide pharmacologiquement acceptable de celle-ci pour la préparation d'un médicament vétérinaire destiné au traitement ou à la prophylaxie d'infections de *Lawsonia* chez les cochons.

2. Utilisation selon la revendication 1, dans laquelle le médicament vétérinaire est un additif pour les aliments ou l'eau de boisson.

3. Utilisation selon la revendication 1 ou 2, dans laquelle le médicament est formulé sous forme d'aliments pour les cochons, contenant de 25 à 200 ppm de 3-O-acétyl-4"-O-isovaléryl-tylosine ou d'un sel d'addition à un acide pharmacologiquement acceptable de celle-ci.

4. Utilisation selon la revendication 3, dans laquelle la concentration en 3-O-acétyl-4"-O-isovaléryl-tylosine dans les aliments est de 40 à 120 ppm.

5. Utilisation selon l'une quelconque des revendications 2 à 4, dans laquelle l'additif pour aliments est formulé sous forme d'une composition enrobée stable sous une forme particulaire enrobée avec de la polyvinylpyrrolidone.

6. Utilisation selon la revendication 1 ou la revendication 2, dans laquelle le médicament est formulé de façon à être administré à des cochons dans leur eau de boisson, à une concentration dans la plage de 25 à 100 g pour 200 litres.

7. Utilisation selon la revendication 1, dans laquelle le médicament est adapté de façon à être administré à des cochons en un dosage dans la plage de 1 à 8 mg/kg de poids corporel/jour.
